# EUROPÄISCHE PATENTSCHRIFT

(11) **EP 1 909 738 B1**
(45) Veröffentlichungstag und Bekanntmachung des Hinweises auf die Patenterteilung: **03.11.2010**
(21) Anmeldenummer: 06792584.2
(22) Anmeldetag: 27.07.2006
(51) Int. Cl.: A61K 8/81, A61Q 1/02, A61Q 1/04, A61Q 1/08

(54) **PRODUKT DER DEKORATIVEN KOSMETIK MIT HOHEM WASSERGEHALT**
DECORATIVE COSMETIC PRODUCT HAVING A HIGH WATER CONTENT
PRODUIT DE COSMETIQUE DECORATIVE PRESENTANT UNE TENEUR ELEVEE EN EAU

(30) Priorität: 28.07.2005 DE 102005036497
(43) Veröffentlichungstag der Anmeldung: 16.04.2008
(73) Patentinhaber: Coty Deutschland GmbH, 55116 Mainz (DE)
(72) Erfinder: BARONE, Salvatore J., Staten Island, New York 10309 (US)
(74) Vertreter: Walter, Wolf-Jürgen
(86) Internationale Anmeldenummer: PCT/EP2006/064736
(87) Internationale Veröffentlichungsnummer: WO 2007/012658

(56) Entgegenhaltungen:
- EP-A2- 1 175 885
- WO-A2-2004/032885
- FR-A- 2 845 287
- US-A- 6 036 947

## Beschreibung

Die Erfindung betrifft Produkte der dekorativen Kosmetik mit hohem Wassergehalt.

Kosmetische Präparate, wie Make-up's, Mascaras, Rouges etc., die zu dekorativen Präparaten gehören, enthalten als Cream-Produkte meist nur 10-35% Wasser und als Fluids meist weniger als 70% Wasser, wobei im letzteren Falls dann Thixotropiermittel und oberflächenaktive Mittel zugegeben werden.

Transferbeständige hochglänzende Lippenstiftzusammensetzungen sind z.B. aus der US 6036947 (Barone et al.) bekannt, die 10-70% eines flüchtigen Lösungsmittels verwenden, d.i. Silicone oder Paraffinkohlenwasserstoffe. Andere transferbeständige kosmetische Zusammensetzungen der WO 97/17058 verwenden Siliconverbindungen wie Organosiloxanharze und Diorganosiloxanpolymere.

Die EP 1 175 885 A2 offenbart Puderzubereitungen mit 5 - 90 % Wasser, einem Hydrokolloid und mindestens einem hydrophoben partikulären Stoff.

Aus der FR 2 845 287 A1 ist eine kosmetische Zusammensetzung mit einem Gelbildner aus drei Komponenten bekannt, der ein Copolymer von Polyacrylamid und Ammoniumacrylat und/oder mindestens ein anionisches Acrylcopolymeres, Lecithin oder Phospholipide pflanzlichen Ursprungs und ein Polyglycerylacylat enthält.

Die WO 2004/032885 betrifft eine wasserbeständige Mascarazusammensetzung mit 42 - 75 % Wasser, Pigmenten, oberflächenaktiven Mitteln und einer Ölphase, die ein Öl oder einen Ester, einen Filmbildner auf Siliconbasis und einen Gelbildner enthält.

In der US 6 036 947 wird ein wasserfreier Lippenstift offenbart, der Cyclomethicone, einen Guarbeth-Ester, Trimethylsiloxysilicat, ein nichtflüchtiges Siliconöl, Siliconwachs und trockene Teilchen umfasst.

Der Erfindung liegt die Aufgabe zugrunde, Produkte der dekorativen Kosmetik bereitzustellen, die einen hohen Wassergehalt haben, die nichttrocknende Lösungsmittel enthalten und bei guter Haftfähigkeit auf der Haut zugleich eine sehr gute Transferbeständigkeit aufweisen.

Die neuen Produkte umfassen 70 bis 92 Gew-% Wasser, 2,0 bis 20 Gew-% Pigmente, 2 bis 8 Gew-% eines Gelbildners mit emulgierenden Eigenschaften, umfassend ein Gemisch aus Natriumacrylat-Copolymeren, hydriertem Polyisobuten, Phospholipiden pflanzlicher Herkunft, einem Polyglycerylacylat und einem Öl. Der Rest bis 100 Gew-% des Produktes sind kosmetische Hilfsstoffe, Trägerstoffe, Wirkstoffe oder Gemische davon, wobei alle Prozentangaben auf das Gesamtgewicht des Produktes bezogen sind, und wobei die Produkte keine "trocknenden" Lösungsmittel enthalten, wie Siliconflüssigkeiten wie Dimethicone, Cyclomethicone usw., keine Paraffinkohlenwasserstoffe wie Pentan, Hexan, Heptan, C₈-₂₀-Isoparaffine oder einwertige Alkohole wie Ethanol, Isopropanol usw.

Der Wassergehalt der Produkte liegt bevorzugt im Bereich von 73-90 Gew-%, insbesondere 84-90 Gew-%. Trotz des sehr hohen Wassergehaltes ist die Stabilität der kosmetischen Formel gewährleistet und neben dem kühlenden Effekt wird eine ausgezeichnete Transferbeständigkeit erreicht. Insbesondere bei Lippenstiften wird ein glänzendes Produkt erhalten, dessen Übertragungs(Transfer)beständigkeit außergewöhnlich gut ist.

Die Kombination des erfindungsgemäß eingesetzten Gelbildners mit dem Wegfall von Lösungsmitteln der Gruppe Silicone, niedere einwertige Alkohole, Paraffinkohlenwasserstoffe führt in Zusammensetzung mit Pigmentanteilen > 2% und hohen Wasseranteilen zu einem sehr stabilen Produkt, z.B. einem Stift, der ohne Wachsanteile sehr fest, lagerstabil, transferstabil, hochglänzend und besonders weich im Auftragen ist, d.h. einem ungewöhnlich anwenderfreundlichen Produkt.

Der Pigmentgehalt der Produkte kann vorteilhaft im Bereich von 3-20 Gew-% liegen, bevorzugter 5-20 Gew-% und insbesondere bevorzugt sind 8-15 Gew-%.

Dabei handelt es sich bevorzugt um ünbehandelte Pigmente.

Der Gehalt des Gelbildners liegt vorteilhaft im Bereich von 3-7 Gew-%, insbesondere 2-5%. Der aus drei Komponenten gebildete Gelbildner enthält ein Natriumacrylat-Copolymeres, das vorteilhaft ein Copolymeres von Natriumacrylat mit Acryloyldimethyltaurat ist. Das Phospholipid in dem Gelbildner ist vorteilhaft ein solches, dessen Herkunft aus pflanzlicher Basis kommt, zum Beispiel aus Sojaöl, Sonnenblumensamenöl, Reisöl und Gemischen davon. Als Phospholipide sind bevorzugt Phosphatidylcholin, Phosphatidylethanolamin, Phosphatidylinositol, Phosphatidylserin, Phosphatidsäure und Lysolecithine sowie Gemische davon.

Das Polyglycerylacylat in dem Gelbildner ist bevorzugt ein Polyglycerylstearat, -distearat oder -linoleat, insbesondere ein Polyglyceryl-10-stearat.

Ein bevorzugter Gelbildner ist DC Odessey® Gel (Lucas Meyer Cosmetic S.A., Thiais, Frankreich), mit dem INCI-Namen Sodium Acrylates Copolymer & Hydrogenated Polyisobutene & Sunflower Phospholipids & Polyglyceryl-10 Stearate & Sunflower Seed Oil.

Das erfindungsgemäße Produkt kann weiterhin kosmetische Hilfs- und Trägerstoffe enthalten, wie sie üblicherweise in solchen Zubereitungen verwendet werden, z.B. Konservierungsmittel, Farbstoffe, Verdickungsmittel, Duftstoffe, Alkohole, Polyole, Ester, einen weiteren Gelbildner, polare und unpolare Öle, Polymere, Copolymere, oberflächenaktive Mittel, Wachse (nicht bevorzugt), Stabilisatoren.

Zu kosmetischen Wirkstoffen, die zusätzlich enthalten sein können, gehören z. B. anorganische und organische Lichtschutzmittel, Radikalfänger, Feuchthaltemittel (nicht bevorzugt), Vitamine, Enzyme, pflanzliche Wirkstoffe, Polymere, Antioxidationsmittel, entzündungswidrige natürliche Wirkstoffe, mit Sauerstoff beladene asymmetrische lamellare Aggregate gemäß WO 94/00109; Aufschlussprodukte von Hefen oder pflanzliche Extrakte.

Pigmente, Pigmentgemische oder Pulver mit pigmentartiger Wirkung oder Farbstoffe können zum Beispiel umfassen Eisenoxide, natürliche Aluminiumsilicate wie Ocker, Titandioxid, Glimmer, Kaolin, manganhaltige Tone wie Umbra und roter Bolus, Calciumcarbonat, Talkum, Glimmer-Titanoxid, Glimmer-Titanoxid-Eisenoxid, Wismutoxychlorid, Nylonkügelchen, Keramikkügelchen, expandierte und nichtexpandierte synthetische Polymerpulver, pulverförmige natürliche organische Verbindungen wie gemahlene Festalgen, gemahlene Pflanzenteile, verkapselte und unverkapselte Getreidestärken.

Als weitere Wirkstoffe eingesetzte Antioxidationsmittel sind beispielsweise Vitamine wie Vitamin C und Derivate davon, beispielsweise Ascorbylacetat, -phosphat und -palmitat, Mägnesiumascorbylphosphat; Vitamin A und Derivate davon; Folsäure und deren Derivate, Vitamin E und deren Derivate, wie Tocopherylacetat; Flavone oder Flavonoide; Aminosäuren, wie Histidin, Glycin, Tyrosin, Tryptophan und Derivate davon; Imidazole wie z.B. cis- oder trans-Urocaninsäure und deren Derivate; Peptide wie D,L-Carnosin, D-Carnosin, L-Carnosin und deren Derivate; Carotinoide und Carotine, wie z.B α-Carotin, β-Carotin, Lycopin; Harnsäure und Derivate davon; α-Hydroxysäuren wie Citronensäure, Milchsäure; Apfelsäure; α-Hydroxyfettsäuren wie Palmitinsäure, Phytinsäure, Lactoferrin; Stilbene und deren Derivate; Mannose und deren Derivate; Liponsäure und deren Derivate, z.B. Dihydroliponsäure; Ferulasäure und deren Derivate; Thiole wie Glutathion, Cystein, und Cystin.

Es ist weiterhin vorteilhaft, als zusätzlichen Wirkstoff wasser- und/oder öllösliche UVA- oder UVB-Filter oder beide zuzusetzen. Zu vorteilhaften öllöslichen UVB-Filtern gehören 4-Aminobenzoesäure-Derivate wie der 4-(Dimethylamino)-benzoesäure-(2-ethylhexyl)ester; Ester der Zimtsäure wie der 4-Methoxyzimtsäure(2-ethylhexyl)ester, Benzophenon-Derivate wie 2-Hydroxy-4-methoxybenzophenon; 3-Benzylidencampher-Derivate wie 3-Benzylidencampher.

Bevorzugte öllösliche UV Filter sind Benzophenone-3, Butyl-Methoxybenzoylmethane, Octyl Methoxycinnamate, Octyl Salicylate, 4-Methylbenzylidene Camphor, Homosalate und Octyl Dimethyl PABA.

Wasserlösliche UVB-Filter sind z.B. Sulfonsäurederivate von Benzophenon oder von 3-Benzylidencampher oder Salze wie das Na- oder K-Salz der 2-Phenylbenzimidazol-5-sulfonsäure.

Zu UVA-Filtern gehören Dibenzoylmethan-Derivate wie 1-Phenyl-4-(4'-isopropylphenyl)propan-1,3-dion, Butyl Methoxybenzoylmethane oder Menthyl Anthranilate.

Besonders bevorzugt sind Benzophenone-3, Butyl Methoxydibenzoylmethane, Octyl Methoxycinnamate, Octyl Salicylate, 4-Methylbenzylidene Camphor, Homosalate, Octocrylene, Ethylhexyl Methoxycinnamate, Isoamyl-p-Methoxycinnamate, Octyl Dimethyl PABA, Ethylhexyltriazone, Diethylhexyl Butamido Triazone, Ethylhexyl Salicylate, Methylene Bis-Benzotriazolyl Tetramethylbutylphenol, Disodium Phenyl Dibenzimidazole Tetrasulfonate, Bis-Ethylhexyloxyphenol Methoxyphenyl Triazine.

Weiterhin bevorzugt als Sonnenschutzfilter sind auch anorganische Pigmente auf Basis von Metalloxiden, wie TiO₂, SiO₂, ZnO, Fe₂O₃, ZrO₂, MnO, Al₂O₃, die auch im Gemisch verwendet werden können.

Besonders bevorzugt als anorganische Pigmente sind agglomerierte Substrate von TiO₂ und/oder ZnO, die einen Gehalt an sphärischen und porösen SiO₂-Teilchen aufweisen, wobei die SiO₂-Teilchen eine Teilchengröße im Bereich von 0,05 µm bis 1,5 µm haben, und neben den SiO₂-Teilche andere anorganische teilchenförmige Stoffe mit sphärischer Struktur vorliegen, wobei die sphärischen SiO₂-Teilchen mit den anderen anorganischen Stoffen definierte Agglomerate mit einer Teilchengröße im Bereich von 0,06 µm bis 5 µm bilden (gemäß WO99/06012).

Als Hilfsstoffe eingesetzte Wachse (nicht bevorzugt) können unter natürlichen pflanzlichen Wachsen, tierischen Wachsen, natürlichen und synthetische Mineralwachsen und synthetischen Wachsen ausgewählt werden. Dazu gehören beispielsweise Carnaubawachs, Candellilawachs, Ozokerit, Bienenwachs, Montanwachs, Wollwachs, Ceresin, Mikrowachse, Hartparaffin, Petrolatum, Siliconwachs, Polyethylenglycol- oder -glycolesterwachse. Die Zugabe von Wachsen ist nicht bevorzugt.

Als Hilfsstoffe eingesetzte Öle können übliche kosmetische Öle sein, wie ein Mineralöl; hydriertes Polyisobuten; synthetisches oder aus Naturprodukten hergestelltes Squalan; kosmetische Ester oder Ether, die verzweigt oder unverzweigt, gesättigt oder ungesättigt sein können; pflanzliche Öle; oder Gemische zweier oder mehrerer davon.

Geeignete Öle sind beispielsweise Mineralöle, Hydrogenated Polyisobuten, Polyisopren, Squalane, Tridecyltrimellitat, Trimethylpropan-triisostearat, Isodecylcitrat, Neopentylglycoldiheptanoat, PPG-15-stearylether sowie vorzugsweise pflanzliche Öle, wie Calendulaöl, Jojobaöl, Avocadoöl, Macadamianußöl, Rizinusöl, Kakaobutter, Kokosnußoil, Maisöl, Baumwollsamenöl, Olivenöl, Palmkernöl, Rapssamenöl, Safloröl, Sesamsamenöl, Sojabohnenöl, Sonnenblumensamenöl, Weizenkeimöl, Traubenkernöl, Kukuinußöl, Distelöl und Gemische davon.

Je nachdem welche Öle ausgewählt werden, werden die kosmetischen Eigenschaften der festen Zusammensetzung beeinflusst, wie Transparenzgrad, Weichheit, Härte, Spreitungswirkung.

Polyole sind ebenfalls mögliche Hilfsstoffe für die erfindungsgemäßen Produkte. Dies sind z.B. Propylenglycol, Dipropylenglycol, Ethylenglycol, Isoprenglycol, Glycerin, Butylenglycole, Sorbitol und Gemische davon. Der Anteil des Polyols liegt im Bereich von 0,1 bis 10 Gew-%.

Geeignete Ester oder Ether von Polyolen sind zum Beispiel Propylene Glycol Dioctanoate, Propylene Glycol Dicaprylate 2,30 Dicaprate, Tridecyl Stearate/Neopentyl Glycol Dicaprylate Dicaprate/Tridecyl Trimellitate, Neopentyl Glycol Dioctanoate, Isopropyl Myristate, Diisopropyl Dimer Dilinoleate, Trimethylpropane Triisostearate, Myristyl Ether, Stearyl Ether, Cetearyl Octanoate, Butyl Ether, Dicaprylyl Ether, Fomblin® HC25.

Zu geeigneten weiteren Gelbildnern gehören Carbomer, Xanthangummi, Carrageenan, Akaziengummi, Guargummi, Silicongummi, Agar-Agar, Alginate und Tylosen, Carboxymethylcellulose, Hydroxyethylcellulose, quaternisierte Cellulose, quaternisierter Guar, bestimmte Polyacrylate, Polyvinylalkohol, Polyvinylpyrrolidon, Montmorillonit.

Die Verwendung des erfindungsgemäßen kosmetischen Präparates in der dekorativen Kosmetik kann z.B. erfolgen als Lippenstift, Konturenstift, Lidschatten, Rouge, Abdeck-Formulierung, Make-up, Grundierung, Mascara, Eye-liner oder Lip-liner. Die Herstellungsweise dieser Formulierungen sind dem Fachmann bekannt.

Ein besonders bevorzugtes Produkt ist ein transferbeständiger, hochglänzender Lippenstift. Der Lippenstift hat einen Wassergehalt von 85-90 Gew-% und ist wachsfrei. In Verbrauchertests wurde ein Lippenstift der vorliegenden Erfindung auf die Lippen von Testpersonen aufgebracht. Nach dem Trocknen wurden die Lippen mit einem Tuch abgetupft. In allen Fällen wurden auf dem Tuch keine Spuren des Lippenstiftes gefunden, d.h. eine hohe Transferbeständigkeit ist gegeben. Weiterhin bestätigten die Testpersonen den einzigartigen hohen Glanz und ein ausgezeichnetes weiches Auftragen.

Die Erfindung soll nachstehend durch Beispiele näher erläutert werden. Alle Angaben erfolgen in Gewichtsprozent, sofern nichts anderes angegeben ist.

### Beispiel 1 Make Up I

| | |
|---|---|
| Wasser | q.s. ad 100 |
| Gelbildner (DC Odessey) | 5,0 |
| Konservierungsmittel | 0,5% |
| Farbpigmente | 3,2 |
| Talkum | 4,3 |

Die Pigmente und Füllstoffe werden gemahlen und dispergiert in Wasser von 80-83 °C. Das separat Erhitzen(etwa 80 °C) Gelierungsmittel wird der Wasserphase unter Rühren zugegeben. Das Rühren wird weiterhin fortgesetzt bis das Produkt bis zur gewünschten Konsistenz nachdickt.

### Beispiel 2 Make Up II

| | |
|---|---|
| Wasser | q.s. ad 100 |
| Konservierungsmittel | 0,3 |
| EDTA | 0,1 |
| Titanium Dioxide | 11,0 |
| Farbpigmente | 2,1 |
| Gelbildner (DC Odessey) | 6,5 |
| Parfüm | 0,2 |
| Butylenglycol | 6,0 |

### Beispiel 3 Lidschatten

| | |
|---|---|
| Wasser | q.s. ad 100 |
| Konservierungsmittel | 0,3 |
| EDTA | 0,1 |
| Gelbildner (DC Odessey) | 5,0 |
| Iron Oxides&Mica&Silane (Pigment) | 8,0 |
| Flamenco Twlight Red | 8,0 |
| Farbpigmente | 0,9 |
| Glycerin (99%) | 0,5 |

### Beispiel 4 Abdeck-Formulierung (Concealer)

| | |
|---|---|
| Wasser | q.s. ad 100 |
| Gelbildner (DC Odessey) | 3,5 |
| Konservierungsmittel | 0,3 |
| Farbpigmente | 3,2 |
| Talkum | 4,3 |

### Beispiel 5 Mascara

| | |
|---|---|
| Wasser | q.s. ad 100 |
| Konservierungsmittel | 0,3 |
| EDTA | 0,1 |
| Farbpigmente | 13,1 |
| Gelbildner (DC Odessey) | 5,0 |
| Butylenglycol | 6,0 |

### Beispiel 6 Lippenstift

| | |
|---|---|
| Wasser | q.s. ad 100 |
| Konservierungsmittel | 0,3 |
| EDTA | 0,1 |
| Mica&Titanium Dioxide&Iron Oxides | 5,0 |
| Gelbildner (DC Odessey) | 5,0 |
| Parfüm | 0,3 |

Die Herstellung entspricht der von Beispiel 1.

### Beispiel 7 Vergleichsversuch

In einer Gruppe von 12 Probandinnen wurde der Lippenstift von Beispiel 6 getestet. Der Lippenstift wurde von jeder Probandin auf die Lippen in üblicherweise Weise aufgetragen. Nach einer Trockenzeit von 2 Minuten wurden die Lippen mit einem weißen Tuch 3-4 mal abgetupft. Danach wurden die weißen Tücher vom Versuchsleiter visuell untersucht. In keinem Falle wurden visuell sichtbare Spuren des Lippenstiftes auf dem Tuch gefunden. Die Probandinnen stellten auf Nachfrage in jedem Fall einen außergewöhnlich hohen Glanz der Lippen nach dem Auftragen fest.

## Patentansprüche

1. Produkt der dekorativen Kosmetik mit hohem Wassergehalt, **dadurch gekennzeichnet, dass** es umfasst
70 bis 92 Gew-% Wasser,
2,0 bis 20 Gew-% Pigmente,
2 bis 8 Gew-% eines Gelbildners mit emulgierenden Eigenschaften, umfassend ein Gemisch aus Natriumacrylat-Copolymere, hydriertem Polyisobuten, Phospholipiden pflanzlicher Herkunft, einem Polyglycerylacylat und einem Öl, sowie
den Rest bis 100 Gew-% kosmetische Hilfsstoffe, Trägerstoffe, Wirkstoffe oder Gemische davon, wobei alle Prozentangaben auf das Gesamtgewicht des Produktes bezogen sind, und wobei das Produkt keine Lösungsmittel der Gruppe enthält, bestehend aus Siliconflüssigkeiten, einwertigen C₁₋₄-Alkoholen, Paraffinkohlenwasserstoffen oder Gemischen davon.

2. Produkt nach Anspruch 1, **dadurch gekennzeichnet, dass** der Wassergehalt im Bereich von 73-90 Gew-% liegt, insbesondere 84-90 Gew-%.

3. Produkt nach Anspruch 1, **dadurch gekennzeichnet, dass** der Pigmentgehalt im Bereich von 5-20 Gew-% liegt, insbesondere 8-15 Gew-%.

4. Produkt nach Anspruch 3, **dadurch gekennzeichnet, dass** die Pigmente unbehandelt sind.

5. Produkt nach Anspruch 1, **dadurch gekennzeichnet, dass** der Gehalt des Gelbildners im Bereich von 2-5 Gew-% liegt.

6. Produkt nach Anspruch 1, **dadurch gekennzeichnet, dass** das Natriumacrylat-Copolymeres ein Copolymeres von Natriumacrylat mit Acryloyldimethyltaurat ist.

7. Produkt nach Anspruch 1, **dadurch gekennzeichnet, dass** das Phospholipid aus Sojaöl, Sonnenblumensamenöl, Reisöl oder Gemischen davon herrührt.

8. Produkt nach Anspruch 1, **dadurch gekennzeichnet, dass** das Polyglycerylacylat ein Polyglycerylstearat, -distearat oder -linoleat ist, insbesondere ein Polyglyceryl-10-stearat.

9. Produkt nach Anspruch 1, **dadurch gekennzeichnet, dass** das Produkt als Lippenstift, Konturenstift, Lidschatten, Rouge, Make-up, Grundierung, Mascara, Eye-liner oder Lip-liner vorliegt.

10. Produkt nach Anspruch 9, **dadurch gekennzeichnet, dass** das Produkt ein transferbeständiger Lippenstift ist.

## Claims

1. Decorative cosmetic product having a high water content, which comprises 70-92 % by weight of water, 2.0-20 % by weight of pigments, 2-8 % by weight of a gel-forming agent with emulsifying properties comprising a mixture of sodium acrylate copolymer, hydrogenated polyisobutene, plant-based phospholipids, a polyglyceryl acylate and an oil,
and the remainder up to 100 % of said product is made up of cosmetic auxiliaries, carriers, active agents or mixtures thereof, all percentages being relative to the product's total weight, and wherein the product does not contain solvents of the group consisting of silicone fluids, monovalent C₁₋₄-alcohols, paraffinic hydrocarbons or mixtures thereof.

2. Product according to claim 1, wherein the content of water is in the range of 73-90 % by weight of water, particularly 84-90 % by weight.

3. Product according to claim 1, wherein the content of pigments is in the range of 5-20 by weight, particularly 8-15 % by weight.

4. Product according to claim 3, wherein the pigments are untreated pigments.

5. Product according to claim 1, wherein the content of the gelling agent is in the range of 2-5 % by weight.

6. Product according to claim 1, wherein the sodium acrylate copolymer is a copolymer of sodium acrylate with acryloyldimethyl taurate.

7. Product according to claim 1, wherein the phospholipid is stemming from soya oil, sunflower seed oil, rice oil or mixtures thereof.

8. Product according to claim 1, wherein the polyglyceryl acylate is a polyglyceryl stearate, polyglyceryl distearate or polyglyceryl linoleate, particularly a polyglyceryl-10 stearate.

9. Product according to claim 1, wherein the product is a lipstick, concealer, eye shadow, rouge, make-up, foundation, mascara, eye-liner or lip-liner.

10. Product according to claim 9, wherein the product is a transfer resistant lipstick.

## Revendications

1. Produit de cosmétique décorative à teneur en eau élevée, **caractérisé en ce qu'**il comprend
de 70 à 92 % en poids d'eau,
de 2,0 à 20 % en poids de pigments,
de 2 à 8 % en poids d'un agent gélifiant ayant des propriétés émulsifiantes, comprenant un copolymère d'acrylate de sodium, du polyisobutène hydrogéné, des phospholipides d'origine végétale, un acylate de polyglycéryle et une huile, et
le reste jusqu'à 100 % en poids est constitué d'adjuvants, véhicules et principes actifs cosmétiques, ou des mélanges de ceux-ci, tous les pourcentages étant rapportés au poids total du produit, et le produit ne contient aucun solvant du groupe constitué par des liquides à base de silicone, des alcools en C1 à C4 monovalents, des hydrocarbures paraffiniques, ou des mélanges de ces substances.

2. Produit selon la revendication 1, **caractérisé en ce que** la teneur en eau va de 73 à 90 % en poids, en particulier de 84 à 90 % en poids.

3. Produit selon la revendication 1, **caractérisé en ce que** la teneur en pigment va de 5 à 20 % en poids, en particulier de 8 à 15 % en poids.

4. Produit selon la revendication 3, **caractérisé en ce que** les pigments sont non traités.

5. Produit selon la revendication 1, **caractérisé en ce que** la teneur en agent gélifiant va de 2 à 5 % en poids.

6. Produit selon la revendication 1, **caractérisé en ce que** le copolymère d'acrylate de sodium est un copolymère d'acrylate de sodium et de diméthyltaurate d'acryloyle.

7. Produit selon la revendication 1, **caractérisé en ce que** le phospholipide est issu de l'huile de soja, l'huile de graines de tournesol, l'huile de riz, ou de mélanges de ces huiles.

8. Produit selon la revendication 1, **caractérisé en ce que** le acylate de polyglycéryle est un stéarate, distéarate ou linoléate de polyglycéryle, en particulier un stéarate de polyglycéryle 10.

9. Produit selon la revendication 1, **caractérisé en ce que** le produit se présente sous la forme de rouge à lèvres, crayon contour des lèvres, ombre à paupières, rouge à joues, maquillage, fond de teint, mascara, eyeliner ou lip liner.

10. Produit selon la revendication 9, **caractérisé en ce que** le produit est un rouge à lèvres anti-transfert.
